# EUROPÄISCHE PATENTANMELDUNG

(11) **EP 4 019 054 A1**
(43) Veröffentlichungstag der Anmeldung: **29.06.2022**
(21) Anmeldenummer: 21216341.4
(22) Anmeldetag: 21.12.2021
(51) Int. Cl.: A61L 9/20, F24F 1/0047, F24F 3/16, A61L 9/16

(54) **VORRICHTUNG ZUR LUFTREINIGUNG**

(30) Priorität: 21.12.2020 DE 202020107437 U
(71) Anmelder: visett GmbH, 41352 Korschenbroich (DE)
(72) Erfinder: RAAD, Michel E., 41352 Korchenbroich (DE)
(74) Vertreter: Paul & Albrecht Patentanwälte PartG mbB

(57) **Zusammenfassung**

Die vorliegende Erfindung betrifft eine Vorrichtung (1) zur Luftreinigung umfassend ein Gehäuse (2), das einen Lufteinlass (3) und einen Luftauslas (4) und einen Luftkanal (5) umfasst, und wenigstens einen insbesondere als UV-C-Strahler ausgebildeten UV-Strahler (7), um die den Luftkanal (5) im Betrieb in einer Strömungsrichtung durchströmende, zu reinigende Luft mit UV-Strahlung zu behandeln und so darin enthaltene Mikroorganismen abzutöten, wobei eine Vorwärmeinrichtung (6) vorgesehen ist, die derart ausgebildet und angeordnet ist, dass den Luftkanal (5) durchströmende Luft mit dieser erwärmt werden kann, bevor sie der Behandlung mit der UV-Strahlung unterzogen wird.

## Beschreibung

Die Erfindung betrifft eine Vorrichtung zur Luftreinigung umfassend ein Gehäuse, das einen Lufteinlass und einen Luftauslas und einen Luftkanal aufweist, und wenigstens einen UV-Strahler, um die den Luftkanal im Betrieb in einer Strömungsrichtung durchströmende, zu reinigende Luft mit UV-Strahlung zu behandeln und so darin enthaltene Mikroorganismen abzutöten.

Er ist bekannt, zur Entkeimung von Luft UV-, insbesondere UV-C-Strahlung zu nutzen. Wenn zu reinigende Luft dieser ausgesetzt wird, können Bakterien, Pilze, Viren bzw. andere darin enthaltene Mikroorganismen abgetötet werden, was aus dem Stand der Technik hinlänglich vorbekannt ist. Die zum Einsatz kommenden UV-, insbesondere UV-C-Strahler sind in den Luftreinigungsvorrichtungen vorbekannter Art in der Regel in dem Luftkanal für die Durchführung der zu behandelnden Luft angeordnet.

Insbesondere im Zusammenhang mit der Übertragung von Viruserkrankungen, wie beispielsweise COVID 19 (SARS-CoV-2), spielen sogenannte Aerosole eine Rolle. Der Hauptübertragungsweg ist die respiratorische Aufnahme virushaltiger Partikel, die beim Sprechen, Husten, Niesen aber auch bereits beim Atmen abgegeben werden. Je nach Größe der Virus enthaltenden Gebilde unterscheidet man zwischen den Tröpfchen mit größerer Abmessung einerseits und den Aerosolen mit geringerer Abmessung andererseits. Man kann Aerosole auch als Mikroorganismen, wie etwa Viren, enthaltende Kleinsttröpfchen bezeichnen. Von Aerosolen spricht man insbesondere, wenn der Durchmesser im Bereich von einigen Nanometern bis zu einigen hundert Mikrometern liegt. Während die größeren Tröpfchen schnell zu Boden sinken können, können sich Aerosole auch über längere Zeit in der Luft halten. Dies gilt ganz besonders, wenn ihr Durchmesser bei bis zu 10 Mikrometern liegt. Dies stellt in geschlossenen Räumlichkeiten ein Problem dar.

Die vorbekannten Luftreinigungsvorrichtungen haben sich prinzipiell bewährt. Es besteht jedoch weiterhin Bedarf an verbesserten Vorrichtungen, insbesondere an solchen, die auch eine Lösung für die Aerosolproblematik bieten.

Es ist daher Aufgabe der vorliegenden Erfindung, eine Vorrichtung der eingangs genannten Art derart weiterzuentwickeln, dass mittels dieser die Aerosolkonzentration in einem geschlossene Raum mit vertretbarem Aufwand zuverlässig in einem akzeptablen Bereich gehalten werden kann.

Diese Aufgabe wird bei einer Vorrichtung der eingangs genannten Art dadurch gelöst, dass eine Vorwärmeinrichtung vorgesehen ist, die derart ausgebildet und angeordnet ist, dass den Luftkanal durchströmende Luft mit dieser erwärmt werden kann, bevor sie der Behandlung mit der UV-Strahlung unterzogen wird.

Der Erfindung basiert mit anderen Worten auf dem Grundgedanken, eine UV-Strahlungsbehandlung mit einer vorgeschalteten Luftvorwärmung zu kombinieren. Wie sich gezeigt hat, kann so auf überraschend einfache Weise ein besonders optimales Reinigungsergebnis erhalten werden. Vor der eigentlichen, konventionellen Entkeimungsbehandlung wird die zu reinigende Luft gezielt vorgewärmt, konkret auf eine höhere Temperatur gebracht, als sie sie bei Eintritt in die erfindungsgemäße Vorrichtung aufweist. Durch das Vorwärmen werden in der zu reinigenden Luft enthaltene Aerosole aufgelöst bzw. ausgetrocknet. Bei der sich anschließenden, zusätzlichen UV-Behandlung werden Mikroorganismen einschließlich Viren auf konventionelle Weise eliminiert. Überraschender Weise hat sich gezeigt, dass eine der UV-Behandlung vorgeschaltete Erwärmung das Entkeimungsergebnis erheblich verbessern kann.

Derzeit geht die Anmelderin davon aus, dass die erfindungsgemäße Vorwärmung eine Reduzierung des Durchmessers der Kleinsttröpfchen darstellenden Aerosole bzw. eine vollständige Entfernung des Feuchtigkeitsanteils dieser bewirkt und die sich unmittelbar anschließenden UV-Behandlung zur Abtötung der Mikroorganismen, wie etwa Viren, aufgrund der reduzierten/fehlenden Feuchtigkeit deutlich effizienter erfolgen kann. Im Ergebnis wird mit vergleichsweise einfachen Mitteln ein hervorragendes Entkeimungsergebnis erzielt. Es wird eine besonders aerosolarme bzw. aerosolfreie, entkeimte Luft erhalten und das Ansteckungsrisiko kann zuverlässig vermieden bzw. reduziert werden.

Auch können aufgrund der Vorwärmung verglichen mit konventionellen Systemen geringere Verweilzeiten der Luft in der UV-Behandlungsstrecke des Luftkanals ausreichen, um ein gleich gutes Entkeimungsergebnis zu erzielen. Die erfindungsgemäße Vorrichtung kann dadurch besonders kompakt ausfallen. Erfindungsgemäße Vorrichtungen können beispielsweise länglich ausgebildet sein und eine Länge im Bereich von 50 cm bis 100 cm, bevorzugt 50 cm bis 80 cm aufweisen.

Die Vorwärmeinrichtung ist derart ausgebildet und angeordnet, dass eine Erwärmung der Luft erfolgt, bevor diese der Entkeimungsbehandlung durch den oder im Falle mehrere die UV-Strahler ausgesetzt wird.

Als besonders geeignet hat es sich erwiesen, wenn sich die Vorwärmeinrichtung zumindest abschnittsweise in dem Luftkanal erstreckt. Die Vorwärmeinrichtung oder Komponenten dieser können insbesondere in dem Luftkanal angeordnet sein.

Weiter bevorzugt ist die Vorwärmeinrichtung derart angeordnet, dass sie im Betrieb zumindest abschnittsweise mit der den Luftkanal durchströmenden, zu reinigenden Luft in Kontakt kommt, insbesondere von der den Luftkanal durchströmenden, zu reinigenden Luft zumindest abschnittsweise beströmt oder umströmt wird. Es kann sein, dass die Anordnung derart getroffen ist, dass die Vorwärmeinrichtung über ihre gesamte Ausdehnung mit der zu reinigenden Luft in Kontakt kommt, beispielsweise die Luft über die gesamte Oberfläche der Vorwärmeinrichtung strömt. Alternativ ist es auch möglich, dass die Vorwärmeinrichtung im Betrieb nur abschnittsweise mit der Luft in Kontakt kommt bzw. gebracht wird, z.B. nur wenigstens eine Komponente dieser, etwa wenigstens ein Heizelement, mit der Luft in Kontakt kommt bzw. gebracht wird, insbesondere von der Luft beströmt bzw. umströmt wird.

Es ist insbesondere vorgesehen, dass die Vorwärmeinrichtung in Strömungseinrichtung beabstandet von dem wenigstens einen UV-Strahler angeordnet ist. Besonders bevorzugt erstreckt sich die Vorwärmeinrichtung zumindest abschnittsweise in Strömungsrichtung vor dem wenigstens einen UV-Strahler.

Der Luftkanal kann einen Vorwärmabschnitt umfassen, in dem die Vorwärmung der Luft mittels der Vorwärmeinrichtung erfolgt, und einen UV-Behandlungsabschnitt, in welchem die Luft mit der UV-Strahlung behandelt wird, um darin enthaltene Mikroorganismen abzutöten, wobei der Vorwärmabschnitt in Strömungsrichtung vor dem UV-Behandlungsabschnitt liegt. Der Luftkanal kann sich durch einen geraden Verlauf auszeichnen. Er kann aber auch zumindest abschnittsweise gebogen verlaufen, mit anderen Worten wenigstens einen gebogenen Abschnitt umfassen.

Die Vorwärmeinrichtung der erfindungsgemäßen Vorrichtung kann prinzipiell auf beliebige Weise ausgebildet sein, solange sie geeignet ist, eine Temperaturerhöhung der den Luftkanal im Betrieb durchströmenden Luft zu bewirken.

Die Vorwärmeinrichtung kann zum Beispiel mit Strom gespeist werden. Dann weist sie zweckmäßiger Weise wenigstens ein Heizelement auf, das im Betrieb durch ohmsche Heizung erwärmt werden kann. Um einen besonders umweltfreundlichen Betrieb zu gewährleisten, kann ein Betrieb mit Strom aus erneuerbaren Energien vorgesehen sein.

Bei dem wenigstens einen Heizelement kann es sich zum Beispiel um wenigstens einen Heizdraht und/oder wenigstens eine Heizwendel handeln.

Es ist auch möglich, dass die Vorwärmeinrichtung mit einem Wärmeträgermedium gespeist wird, welches an einem von der Vorwärmeinrichtung, gegebenenfalls der gesamten erfindungsgemäßen Vorrichtung separaten Ort auf eine vorgegebene Mindesttemperatur wird. Dann weist die Vorwärmeinrichtung zweckmäßiger Weise wenigstens einen Strömungskanal für ein Wärmeträgermedium auf. Dies kann alternativ oder zusätzlich dazu vorgesehen sein, dass die erfindungsgemäße Vorrichtung wenigstens ein Heizelement umfasst, das im Betrieb durch ohmsche Heizung erwärmt werden kann.

Auch wenn es in der Regel ausreichen wird, dass das Gehäuse einen Lufteinlass und einen Luftauslass aufweist, ist nicht ausgeschlossen, dass mehr als ein Lufteinlass und/oder mehr als ein Luftauslass vorgesehen sind.

Der oder der jeweilige in dem Gehäuse vorgesehene Lufteinlass dient dem Einlass von zu reinigender Luft in das Gehäuse und insbesondere den darin vorgesehenen Luftkanal. Durch den oder den jeweiligen in dem Gehäuse vorgesehenen Luftauslass kann gereinigte Luft wieder aus dem Gehäuse austreten. Zweckmäßiger Weise ist der Luftkanal sowohl mit dem bzw. im Falle mehrerer mit dem jeweiligen Lufteinlass als auch mit dem bzw. im Falle mehrere dem jeweiligen Luftauslass fluidtechnisch verbunden, so dass Luft durch den (jeweiligen) Einlass in das Gehäuse eintreten kann, in den Luftkanal strömt, dort zunächst vorgewärmt und dann mit der UV-Strahlung behandelt wird, und im gereinigten Zustand zu dem (jeweiligen) Luftauslass gelangt und das Gehäuse wieder verlässt.

Der UV-Strahler ist bzw. im Falle mehrere die UV-Strahler sind besonders bevorzugt als UV-C-Strahler ausgebildet. Strahlung aus diesem Wellenlängenbereich hat sich als besonders geeignet zum Abtöten von in der Luft enthaltenen Mikroorganismen erwiesen. Unter UV-Strahlung ist insbesondere solche aus dem Wellenlängenbereich von 100 Nanometern bis 380 Nanometern zu verstehen, unter UV-C-Strahlung insbesondere solche aus dem Wellenlängenbereich von 100 Nanometern bis 280 Nanometern. Bevorzugt ist der wenigstens eine UV-Strahler ausgebildet, um UV-C-Licht einer Wellenlänge von 253,7 Nanometern zu emittieren. Diese Wellenlänge ist für die Entkeimung besonders geeignet. Mikroorganismen werden mit Licht dieser Wellenlänge besonders gut eliminiert. Bei dem wenigstens einen UV-Strahler kann es sich beispielsweise um einen UV-Niederdruckstrahler handeln.

Es sei angemerkt, dass die erfindungsgemäße Vorrichtung, insbesondere deren Gehäuse bevorzugter Weise derart ausgestaltet bzw. ausgestattet ist, dass ein Austreten von UV-Strahlung, die im Betrieb von dem oder den UV-Strahlern emittiert wird, nicht möglich ist. Rein beispielhaft sei genannt, dass die Vorrichtung eine oder mehrere Lichtfallen umfasst, die z.B. an oder nahe des Lufteinlasses und/oder an oder nahe des Luftauslasses angeordnet sind, und durch die zwar Luft hindurchströmen kann, durch welche jedoch keine von den UV-Strahlern abgestrahlte UV-Strahlung nach außen gelangen kann. So wird ein besonders sicherer Betrieb der Vorrichtung gewährleistet.

Die erfindungsgemäße Vorrichtung umfasst zweckmäßiger Weise wenigsten eine Luftfördereinrichtung zur Förderung von Luft in und durch den in dem Gehäuse vorgesehenen Luftkanal. Die wenigstens eine Luftfördereinrichtung kann in dem Gehäuse, insbesondere in dem Luftkanal angeordnet sein. Die wenigstens eine Luftfördereinrichtung kann beispielsweise durch einen Ventilator gegeben sein oder einen solchen umfassen.

Es hat sich gezeigt, dass eine Austrocknung der Aerosole besonders zuverlässig erfolgt, wenn die Lufttemperatur mittels der Vorwärmeinrichtung um wenigstens 8°C, bevorzugt um wenigstens 15°C erhöht wird.

Eine weitere ganz besonders bevorzugte Ausgestaltung der erfindungsgemäßen Vorrichtung zeichnet sich entsprechend dadurch aus, dass diese eine mit der Vorwärmeinrichtung verbundene Steuer- oder Regeleinrichtung umfasst, die ausgebildet und eingerichtet ist, um die Temperatur der den Luftkanal durchströmenden Luft um wenigstens 8°C, bevorzugt wenigstens 15°C zu erhöhen. Die Temperaturerhöhung in dem genannten Bereich erfolgt dabei zweckmäßiger Weise gegenüber der Eintrittstemperatur, die die Luft aufweist, wenn sie in die erfindungsgemäße Vorrichtung eintritt. Rein beispielhaft sei genannt, dass Luft mit einer Temperatur von 20°C bzw. 21°C in die Vorrichtung eintritt. Dann würde eine Temperaturerhöhung auf mindesten 28°C bzw. mindestens 29°C, bevorzugt mindestens 35°C bzw. mindestens 36°C erfolgen.

Als ganz besonders geeignet hat es sich erwiesen, wenn eine Vorwärmung der Luft auf eine Temperatur im Bereich von 28°C bis 50°C, insbesondere 30°C bis 50°C, bevorzugt 35°C bis 50°C erfolgt.

Eine weitere ganz besonders bevorzugte Ausgestaltung der erfindungsgemäßen Vorrichtung zeichnet sich entsprechend dadurch aus, dass diese eine mit der Vorwärmeinrichtung verbundene Steuer- oder Regeleinrichtung umfasst, die ausgebildet und eingerichtet ist, um die den Luftkanal durchströmende Luft auf eine Temperatur im Bereich von 28°C bis 50°C, insbesondere 30°C bis 50°C, bevorzugt 35°C bis 50°C zu erwärmen und insbesondere zu halten.

Sind eine Steuer- oder Regeleinrichtung und wenigstens eine Luftfördereinrichtung vorhanden, ist in bevorzugter Weiterbildung ferner vorgesehen, dass die Steuer- oder Regeleinrichtung mit der wenigstens einen Luftfördereinrichtung verbunden und derart ausgebildet und eingerichtet ist, dass sie die Vorwärmeinrichtung und/oder die wenigstens eine Luftförderrichtung derart steuert oder regelt, dass die den Luftkanal durchströmende Luft auf die Temperatur im Bereich von 28°C bis 50°C, insbesondere 30°C bis 50°C, bevorzugt 35°C bis 50°C erwärmt und insbesondere auf dieser Temperatur gehalten wird.

Es kann auch sein, dass die Vorrichtung eine Temperaturmesseinrichtung aufweist, um die Temperatur der den Luftkanal durchströmenden Luft zu erfassen. Zweckmäßigerweise ist die wenigstens eine Temperaturmesseinrichtung dann derart ausgebildet und angeordnet, dass mit dieser die Lufttemperatur in dem Luftkanal in einem Bereich erfasst werden kann, in dem die Luft bereits der Vorwärmbehandlung mittels der Vorwärmeinrichtung unterzogen wurde. Die Temperaturmesseinrichtung kann derart ausgebildet und angeordnet sein, dass mit dieser die Lufttemperatur in dem Luftkanal in einem Bereich erfasst werden kann, der in Strömungsrichtung vor dem wenigstens einen UV-Strahler liegt. Sie kann insbesondere derart ausgebildet und angeordnet sein, dass mit dieser die Lufttemperatur in dem Luftkanal in einem Bereich erfasst werden kann, der zwischen der Vorwärmeinrichtung und dem wenigstens UV-Strahler liegt.

Die Temperaturmesseinrichtung kann beispielsweise einen oder mehrere Temperatursensoren oder dergleichen aufweisen oder durch einen oder mehrere Temperatursensoren oder dergleichen gegeben sein.

Ist eine Temperaturmesseinrichtung vorgesehen, gilt weiter bevorzugt, dass die Steuer- oder Regeleinrichtung mit der Temperaturmesseinrichtung verbunden und derart ausgebildet und eingerichtet ist, dass sie die mit der wenigstens einen Luftfördereinrichtung geförderte Luftmenge und/oder die Heizleistung der Vorwärmeinrichtung in Abhängigkeit der mit der Temperaturmesseinrichtung erfassten Temperatur regelt, dies zweckmäßiger Weise derart, dass eine Erwärmung der den Luftkanal durchströmenden Luft um wenigstens 8°C, bevorzugt wenigstens 15°C erfolgt bzw. Derart, dass eine Temperatur im vorgenannten Temperaturbereich erhalten wird, also eine Temperatur im Bereich von 28°C bis 50°C, insbesondere 30°C bis 50°C, bevorzugt 35°C bis 50°C .

Es ist auch möglich, dass eine Luftfeuchtigkeitsmesseinrichtung vorgesehen ist, um die Luftfeuchtigkeit der im Betrieb in die Vorrichtung eintretenden Luft zu messen. Eine solche kann beispielsweise an oder nahe des Lufteinlasses angeordnet sein, um die in das Gehäuse eintretende Luft zu messen.

Ist eine Luftfeuchtigkeitsmesseinrichtung vorhanden, kann weiterhin vorgesehen sein, dass die Steuer- oder Regeleinrichtung mit der Luftfeuchtigkeitsmesseinrichtung verbunden und derart ausgebildet und eingerichtet ist, dass sie die mit der wenigstens einen Luftfördereinrichtung geförderte Luftmenge und/oder die Heizleistung der Vorwärmeinrichtung in Abhängigkeit der mit der Luftfeuchtigkeitsmesseinrichtung erfassten Luftfeuchtigkeit anpasst, insbesondere steuert. Mit anderen Worten kann eine Anpassung in Abhängigkeit der Luftfeuchtigkeit der zugeführten, zu reinigenden Luft erfolgen. Beispielsweise kann in Reaktion auf die messtechnische Erfassung eine Erhöhung der Feuchtigkeit der der Vorrichtung zugeführten, zu reinigenden Luft die Heizleistung der Vorwärmeinrichtung erhöht und/oder die mit der Luftfördereinrichtung geförderte Luftmenge reduziert werden.

Weitere Merkmale und Vorteile der vorliegenden Erfindung werden anhand der nachfolgenden Beschreibung von Ausführungsformen des erfindungsgemäßen Verfahrens und erfindungsgemäßer Systeme unter Bezugnahme auf die beiliegende Zeichnung deutlich. Darin zeigt die einzige Figur ein Ausführungsbeispiel einer erfindungsgemäßen Vorrichtung 1 zur Luftreinigung in rein schematischer, stark vereinfachter Schnittdarstellung.

Die Vorrichtung 1 ist einem nicht weiter dargestellten Innenraum angeordnet, und dient im Betrieb der Reinigung der Raumluft.

Die Vorrichtung 1 umfasst ein Gehäuse 2 mit einem Lufteinlass 3 und einem Luftauslass 4, die bei dem gezeigten Beispiel an einander gegenüberliegenden Stirnseiten des Gehäuses 2 vorgesehen sind. Das Gehäuse 2 ist hier quaderförmig ausgebildet, wobei dies rein beispielhaft zu verstehen ist und prinzipiell auch andere Gehäuseformen möglich sind. Durch den Lufteinlass 3 kann zu reinigende Raumluft in das Gehäuse 2 eintreten. Durch den Luftauslass 4 kann gereinigte Luft das Gehäuse 2 wieder verlassen und dem Raum zugeführt werden. Die Lufteinlass- und Luftauslassrichtung ist in der Figur durch Pfeile angedeutet. Es sei angemerkt, dass es möglich ist, dass das Gehäuse 2 einer erfindungsgemäßen Vorrichtung 1 mehr als einen Lufteinlass und/oder mehr als einen Luftauslass 4 aufweist. Rein beispielhaft sei genannt, dass an den beiden gegenüberliegenden, in der Figur nach links und rechts weisenden Gehäuseseiten jeweils noch ein zweiter Lufteinlass und/oder Luftauslass vorgesehen ist, der sich z.B. neben dem dargestellten Ein- bzw. Auslass 3, 4 befinden kann.

Das Gehäuse 2 umfasst einen Luftkanal 5, der in dem Gehäuseinneren definiert ist. In dem Luftkanal 5, der im Betrieb von zu reinigender Luft durchströmt wird, erfolgt die Luftreinigung. Der Luftkanal 5 ist hier länglich und zeichnet sich durch einen geraden Verlauf aus, wobei auch dies rein beispielhaft zu verstehen ist. Eine erfindungsgemäße Vorrichtung 1 kann natürlich auch einen Luftkanal 5 umfassen kann, der zumindest abschnittsweise einen gekrümmten bzw. gebogenen Verlauf aufweist. In dem Gehäuse 2 können eines oder mehrere Luftleitbleche angeordnet sein, welche den Luftkanal 5 definieren, mit anderen Worten begrenzen. Der Luftkanal 5 ist sowohl mit der Lufteinlassöffnung 2 als auch der Luftauslassöffnung 4 fluidtechnisch verbunden.

Die erfindungsgemäße Vorrichtung 1 umfasst weiterhin eine Vorwärmeinrichtung 6, mehrere UV-Strahler 7 und eine bei dem dargestellten Beispiel durch einen Ventilator 8 gegebene Luftfördereinrichtung. Die Vorwärmeinrichtung 6, die UV-Strahler 7 und der Ventilator 8 sind hier in dem Gehäuse 2 der Vorrichtung 1, konkret in dem Luftkanal 5 dieser angeordnet.

Die Vorwärmeinrichtung 6 ist derart ausgebildet und angeordnet, dass eine Erwärmung der Luft erfolgt, bevor diese der Entkeimungsbehandlung die UV-Strahler 7 ausgesetzt wird.

Die Vorwärmeinrichtung 6 ist derart in dem Luftkanal 4 angeordnet, dass sie im Betrieb mit der den Luftkanal 5 durchströmenden, zu reinigenden Luft in Kontakt kommt, konkret zumindest ein Abschnitt dieser von der den Luftkanal 5 durchströmenden Luft umströmt wird. Die Vorwärmeinrichtung 6 umfasst hier mehrere Heizelemente in Form von Heizdrähten oder Heizwendeln, die im Betrieb durch ohmsches Heizen erwärmt werden und über welche die Luft geführt und so erwärmt wird. Es sei betont, dass eine Vorwärmeinrichtung 6 mit strombetriebenen Heizelementen nur ein geeignetes Beispiel darstellt und aus beliebige andere Ausgestaltungen möglich sind, sofern sie eine Lufterwärmung ermöglichen.

Wie man sieht, ist die Vorwärmeinrichtung 6 den UV-Strahlern 7 in der durch die Pfeile angedeuteten Strömungsrichtung vorgeschaltet, so dass zu reinigende Luft, die den Luftkanal 5 im Betrieb in Strömungsrichtung durchströmt, erst die Vorwärmeinrichtung 6 und dann die UV-Strahler 7 erreicht. Die Vorwärmeinrichtung 6 ist mit anderen Worten in Strömungsrichtung beabstandet und vor den UV-Strahlern 7 angeordnet.

Bei den UV-Strahlern 7 handelt es sich vorliegend um UV-C-Strahler, die im Betrieb Licht aus dem UV-C-Wellenlängenbereich emittieren. Die UV-C-Strahler 7 sind hier durch röhrenförmige UV-Niederdruckstrahler gegeben, die UV-C-Licht einer Wellenlänge von 253,7 Nanometern emittieren. Diese Wellenlänge ist für die Entkeimung besonders geeignet.

Der Ventilator 8 ist in Strömungsrichtung hinter den UV-C-Strahlern 7 und vor dem Luftaustritt 4 angeordnet.

Das dargestellte Beispiel einer erfindungsgemäßen Vorrichtung umfasst darüber hinaus eine durch eine Temperatursensor 9 gegebene Temperaturmesseinrichtung und eine Regeleinrichtung 10. Der Temperatursensor 9 ist derart ausgebildet und angeordnet, dass mit diese die Lufttemperatur in dem Luftkanal 5 in einem Bereich zwischen der Vorwärmeinrichtung 6 und den UV-C-Strahlern 7 erfasst werden kann, mit anderen Worten in einem Bereich, wo die den Luftkanal 5 im Betrieb durchströmende Luft bereits der Vorwärmbehandlung mittels der Vorwärmeinrichtung 6 unterzogen wurde, mit anderen Worten vorgewärmt ist, und bevor sie die UVC-Strahler 7 erreicht.

Die Regeleinrichtung 10 ist nicht nur mit dem Temperatursensor 9 verbunden, sondern darüber hinaus auch mit der Vorwärmeinrichtung 6 und dem die Luftfördereinrichtung bildenden Ventilator 8.

Mit Hilfe der Regeleinrichtung 10 wird die Lufttemperatur im Betrieb um wenigstens 8°C, bevorzugt wenigstens 15°C erhöht. Bei dem dargestellten Ausführungsbeispiel wird mit Hilfe der Regeleinrichtung 10 die Lufttemperatur konkret auf eine Temperatur in einem gewünschten Bereich gebracht und insbesondere gehalten, bevorzugt eine Temperatur im Bereich von 35°C bis 50°C.

Die Regeleinrichtung 10 ist hierfür derart ausgebildet und eingerichtet, dass sie die Heizleistung der Vorwärmeinrichtung 6 und die mit dem Ventilator 8 geförderte Luftmenge in Abhängigkeit von der mittels des Temperatursensors 9 erfassten Temperaturwertes regelt und zwar derart, dass die den Luftkanal 5 durchströmende Luft auf eine Temperatur in diesem Bereich, also 35°C bis 50°C gebracht und gehalten wird.

Es sei betont, dass, auch wenn die Regeleinrichtung 10 bei dem dargestellten Beispiel mit der Vorwärmeinrichtung 6 und dem Ventilator 8 verbunden ist und beide Komponenten im Betrieb steuert bzw. regelt, dies nicht zwingend der Fall sein muss. Vielmehr wäre es prinzipiell auch möglich, dass die Vorwärmeinrichtung 6 nur eine konstante, nicht veränderbare Heizleistung erbringt oder der Ventilator 8 nur eine konstante, nicht veränderbare Luftmenge fördert, etwa nur eine Drehgeschwindigkeit hat, und dann nur die jeweils andere Komponente geregelt wird.

Im Folgenden wird die Funktionsweise des dargestellten Ausführungsbeispiels einer erfindungsgemäßen Vorrichtung 1 näher erläutert.

Im Betrieb der Vorrichtung 1 ist der Ventilator 8 aktiviert und es wird zu reinigende Luft durch den Lufteinlass 3 in das Gehäuse 2, durch den Luftkanal 5 zu dem Luftauslass 4 gefördert und tritt dort wieder aus dem Gehäuse 2 aus.

In dem Luftkanal 5 erfolgt zunächst eine Vorwärmung der zu reinigenden Luft mittels der im Betrieb ebenfalls aktivierten Vorwärmeinrichtung 6. Der Abschnitt des Luftkanals 5, in dem die Vorwärmeinrichtung 6 bzw. die Heizdrähte dieser angeordnet sind und die Vorwärmbehandlung erfolgt, kann auch als Vorwärmabschnitt 5a des Luftkanals 5 bezeichnet werden. Konkret wird von den im Betrieb erwärmten Heizdrähten der Vorwärmeinrichtung 6 Wärme an die an diesen vorbeiströmende Luft abgegeben. Dabei wird die Heizleistung der Vorwärmeinrichtung 6 und die Drehzahl des Ventilators 8 von der Regeleinrichtung 10 derart geregelt, dass sich in demjenigen Bereich des Luftkanals 5, in dem der Temperatursensor 9 vorgesehen ist, eine Lufttemperatur im Bereich von 35°C bis 50°C einstellt und diese gehalten wird. Es wird beispielsweise auf eine Temperatur von 33°C oder 35°C geregelt.

Es sei angemerkt, dass es auch möglich ist, die Luftfeuchtigkeit der zu reinigenden Luft zu messen und dies bei der Regelung zu berücksichtigen, insbesondere in Abhängigkeit dieser eine Anpassung der Heizleistung der Vorwärmeinrichtung 6 und/oder der Luftfördermenge des Ventilators 8 vorzunehmen. Hierzu kann die erfindungsgemäße Vorrichtung 1 eine mit der Regeleinrichtung 10 verbundene Luftfeuchtigkeitsmesseinrichtung 11 umfassen.

Eine solche kann beispielsweise in dem Gehäuse 2 unmittelbar an oder hinter dem Lufteinlass 3 angeordnet sein, wie beispielhaft in der Figur gezeigt, oder auch außenseitig an dem Gehäuse 2 nahe des Lufteinlasses 3.

Nachdem die Luft der Vorwärmbehandlung mittels der Vorwärmeinrichtung 6 unterzogen wurde, erreicht sie die UV-C-Strahler 7 und erfährt eine Entkeimungsbehandlung. Der Abschnitt des Luftkanals 5, in dem die UV-Strahler 7 angeordnet sind und die UV-Behandlung der Luft erfolgt, kann auch als UV-Behandlungsabschnitt 5b des Luftkanals 5 bezeichnet werden. Die von den UV-C-Strahlern 7 emittierte UV-C-Strahlung trifft hier auf in der Luft enthaltene Mikroorganismen, insbesondere Viren, Bakterien und Pilze, und tötet diese ab, so dass entkeimte Luft erhalten wird.

Aufgrund der erfindungsgemäßen Kombination von Vorwärmung und anschließender UV-Behandlung wird auf überraschend einfache Weise ein besonders optimales Reinigungsergebnis erzielt. Derzeit geht die Anmelderin davon aus, dass die Vorwärmung eine Reduzierung des Durchmessers der Kleinsttröpfchen darstellenden Aerosole bzw. eine vollständige Entfernung des Feuchtigkeitsanteils dieser bewirkt und die sich unmittelbar anschließenden UV-Behandlung zur Abtötung der Mikroorganismen, wie etwa Viren, aufgrund der reduzierten/fehlenden Feuchtigkeit deutlich effizienter erfolgen kann. Im Ergebnis wird mit vergleichsweise einfachen Mitteln ein hervorragendes Entkeimungsergebnis erzielt. Es wird eine besonders aerosolarme bzw. aerosolfreie, entkeimte Luft erhalten und das Ansteckungsrisiko kann zuverlässig vermieden bzw. reduziert werden.

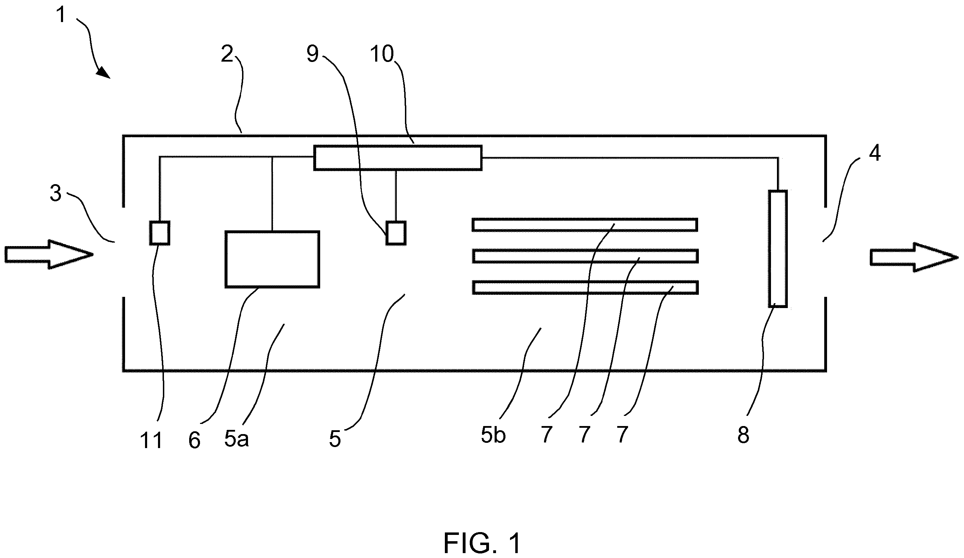

Auch können aufgrund der erfindungsgemäßen Vorwärmung verglichen mit konventionellen Systemen geringere Verweilzeiten der Luft in der UV-Behandlungsstrecke 5b des Luftkanals 5 ausreichen, um ein gleich gutes Entkeimungsergebnis zu erzielen. Die erfindungsgemäße Vorrichtung 1 kann dadurch besonders kompakt ausfallen. Die Länge einer erfindungsgemäßen Vorrichtung 1 (entspricht bei dem Beispiel aus Figur 1 der Ausdehnung in Luftströmungsrichtung, also in Richtung der Pfeile) kann insbesondere im Bereich von n50 cm bis 100 cm, bevorzugt 50 cm bis 80 cm liegen.

Es sei angemerkt, dass die Vorrichtung 1, insbesondere deren Gehäuse 2, bevorzugter Weise derart ausgestaltet bzw. ausgestattet ist, dass ein Austreten von UV-Strahlung, die im Betrieb von den UV-Strahlern 7 emittiert wird, zuverlässig verhindert wird. Rein beispielhaft sei genannt, dass die Vorrichtung 1 eine oder mehrere Lichtfallen umfasst, die z.B. an oder nahe des Lufteinlasses 3 und/oder an oder nahe des Luftauslasses 4 angeordnet sind, und durch die zwar Luft hindurchströmen jedoch keine von den UV-Strahlern 7 abgestrahlte UV-Strahlung nach außen gelangen kann. So wird ein besonders sicherer Betrieb der Vorrichtung 1 gewährleistet.

## Patentansprüche

1. Vorrichtung (1) zur Luftreinigung umfassend ein Gehäuse (2), das einen Lufteinlass (3) und einen Luftauslas (4) und einen Luftkanal (5) umfasst, und wenigstens einen insbesondere als UV-C-Strahler ausgebildeten UV-Strahler (7), um die den Luftkanal (5) im Betrieb in einer Strömungsrichtung durchströmende, zu reinigende Luft mit UV-Strahlung zu behandeln und so darin enthaltene Mikroorganismen abzutöten, **dadurch gekennzeichnet, dass** eine Vorwärmeinrichtung (6) vorgesehen ist, die derart ausgebildet und angeordnet ist, dass den Luftkanal (5) durchströmende Luft mit dieser erwärmt werden kann, bevor sie der Behandlung mit der UV-Strahlung unterzogen wird.

2. Vorrichtung (1) nach Anspruch 1, **dadurch gekennzeichnet, dass** sich die Vorwärmeinrichtung (6) zumindest abschnittsweise in dem Luftkanal erstreckt, wobei, insbesondere, die Vorwärmeinrichtung (6) in Strömungseinrichtung beabstandet von dem wenigstens einen UV-Strahler (7) angeordnet ist.

3. Vorrichtung (1) nach einem der vorhergehenden Ansprüche, **dadurch gekennzeichnet, dass** die Vorwärmeinrichtung (6) derart angeordnet ist, dass sie im Betrieb mit der den Luftkanal durchströmenden, zu reinigenden Luft zumindest abschnittsweise in Kontakt kommt, insbesondere von der den Luftkanal (5) durchströmenden, zu reinigenden Luft zumindest abschnittsweise beströmt oder umströmt wird.

4. Vorrichtung (1) nach einem der vorhergehenden Ansprüche, **dadurch gekennzeichnet, dass** die Vorwärmeinrichtung (6) mittels Strom betrieben wird.

5. Vorrichtung (1) nach Anspruch 4, **dadurch gekennzeichnet, dass** die Vorwärmeinrichtung (6) wenigstens ein Heizelement, insbesondere wenigstens einen Heizdraht und/oder wenigstens eine Heizwendel, aufweist, das im Betrieb durch ohmsche Heizung erwärmt werden kann.

6. Vorrichtung (1) nach den Ansprüchen 3 bis 5, **dadurch gekennzeichnet, dass** die Vorwärmeinrichtung (6) derart angeordnet ist, dass das wenigstens eine Heizelement im Betrieb mit der den Luftkanal durchströmenden, zu reinigenden Luft zumindest abschnittsweise in Kontakt kommt, insbesondere von der den Luftkanal (5) durchströmenden, zu reinigenden Luft zumindest abschnittsweise beströmt oder umströmt wird.

7. Vorrichtung (1) nach einem der vorhergehenden Ansprüche, **dadurch gekennzeichnet, dass** wenigstens eine Luftfördereinrichtung (8) zur Förderung von Luft in und/oder durch den in dem Gehäuse (2) vorgesehenen Luftkanal (5) vorgesehen ist, bevorzugt, wobei die wenigstens eine Luftförderrichtung (8) durch einen Ventilator gegeben ist oder einen solchen umfasst.

8. Vorrichtung (1) nach einem der vorhergehenden Ansprüche, **dadurch gekennzeichnet, dass** die Vorrichtung (1) eine mit der Vorwärmeinrichtung (6) verbundene Steuer- oder Regeleinrichtung (10) umfasst, die ausgebildet und eingerichtet ist, um die Temperatur der den Luftkanal (5) durchströmenden Luft um wenigstens 8°C, bevorzugt wenigstens 15°C zu erhöhen.

9. Vorrichtung (1) nach Anspruch 7 und 8, **dadurch gekennzeichnet, dass** die Steuer- oder Regeleinrichtung (10) mit der wenigstens einen Luftfördereinrichtung (8) verbunden und derart ausgebildet und eingerichtet ist, dass sie die Vorwärmeinrichtung (6) und/oder die wenigstens eine Luftförderrichtung (8) derart steuert oder regelt, dass die Temperatur der den Luftkanal (5) durchströmenden Luft um wenigstens 8°C, bevorzugt wenigstens 15°C erhöht wird.

10. Vorrichtung (1) nach einem der vorhergehenden Ansprüche, **dadurch gekennzeichnet, dass** die Vorrichtung (1) eine mit der Vorwärmeinrichtung (6) verbundene Steuer- oder Regeleinrichtung (10) umfasst, die ausgebildet und eingerichtet ist, um die den Luftkanal (5) durchströmende Luft auf eine Temperatur im Bereich von 28°C bis 50°C, insbesondere 30°C bis 50°C, bevorzugt 35°C bis 50°C zu erwärmen.

11. Vorrichtung (1) nach Anspruch 8 und 10 **dadurch gekennzeichnet, dass** die Steuer- oder Regeleinrichtung (10) mit der wenigstens einen Luftfördereinrichtung (8) verbunden und derart ausgebildet und eingerichtet ist, dass sie die Vorwärmeinrichtung (6) und/oder die wenigstens eine Luftförderrichtung (8) derart steuert oder regelt, dass die den Luftkanal (5) durchströmende Luft auf die Temperatur im Bereich von 28°C bis 50°C, insbesondere 30°C bis 50°C, bevorzugt 35°C bis 50°C erwärmt wird.

12. Vorrichtung (1) nach einem der vorhergehenden Ansprüche, **dadurch gekennzeichnet, dass** eine Temperaturmesseinrichtung (9) vorgesehen ist, um die Temperatur der den Luftkanal (5) durchströmenden Luft zu erfassen.

13. Vorrichtung (1) nach einem der Ansprüche 8 bis 11 und Anspruch 12, **dadurch gekennzeichnet, dass** die Steuer- oder Regeleinrichtung (10) mit der Temperaturmesseinrichtung (9) verbunden und derart ausgebildet und eingerichtet ist, dass sie die mit der wenigstens einen Luftfördereinrichtung (8) geförderte Luftmenge und/oder die Heizleistung der Vorwärmeinrichtung (6) in Abhängigkeit der mit der Temperaturmesseinrichtung (9) erfassten Temperatur regelt.

14. Vorrichtung (1) nach einem der vorhergehenden Ansprüche, **dadurch gekennzeichnet, dass** eine Luftfeuchtigkeitsmesseinrichtung (11) vorgesehen ist, um die Luftfeuchtigkeit der im Betrieb in die Vorrichtung eintretenden Luft zu messen.

15. Vorrichtung (1) nach einem der Ansprüche 8, 9, 10, 12 oder 13 und Anspruch 14, **dadurch gekennzeichnet, dass** die Steuer- oder Regeleinrichtung (10) mit der Luftfeuchtigkeitsmesseinrichtung (11) verbunden und derart ausgebildet und eingerichtet ist, dass sie die mit der wenigstens einen Luftfördereinrichtung (8) geförderte Luftmenge und/oder die Heizleistung der Vorwärmeinrichtung (6) in Abhängigkeit der mit der Luftfeuchtigkeitsmesseinrichtung erfassten Luftfeuchtigkeit anpasst.
